(19) Europäisches Patentamt
European Patent Office
Office européen des brevets

(11) Veröffentlichungsnummer : **0 233 279 B1**

(12) **EUROPÄISCHE PATENTSCHRIFT**

(45) Veröffentlichungstag der Patentschrift : **11.03.92 Patentblatt 92/11**

(51) Int. Cl.$^5$ : **A61K 31/54**

(21) Anmeldenummer : **86905788.5**

(22) Anmeldetag : **19.09.86**

(86) Internationale Anmeldenummer : **PCT/EP86/00545**

(87) Internationale Veröffentlichungsnummer : **WO 87/01591 26.03.87 Gazette 87/07**

# (54) NEUARTIGE VERWENDUNG VON TAUROLIN.

(30) Priorität : **20.09.85 DE 3533612**

(43) Veröffentlichungstag der Anmeldung : **26.08.87 Patentblatt 87/35**

(45) Bekanntmachung des Hinweises auf die Patenterteilung : **11.03.92 Patentblatt 92/11**

(84) Benannte Vertragsstaaten : **AT BE CH DE FR GB IT LI LU NL SE**

(56) Entgegenhaltungen :
**FR-A- 2 455 890**
**TAUROLIN (Wil. Brückner & R.W. Pfirrmann) (1985), Urban & Schwarzenbers, München, S. 20 n. 30**

(56) Entgegenhaltungen :
**Forth, Henschler, Rummel: Allgem. u. spezielle Pharmakologie, Mannheim (1977), S. 152-154**
**PSCHYREMBEL, KLIN. WOERTERBUCH, Berlin (1977), S. 13-14**

(73) Patentinhaber : **Reinmüller, Johannes, Dr. med.**
**Gustav-Freytag-Strasse 27**
**W-6200 Wiesbaden (DE)**

(72) Erfinder : **Reinmüller, Johannes, Dr. med.**
**Gustav-Freytag-Strasse 27**
**W-6200 Wiesbaden (DE)**

(74) Vertreter : **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86 (DE)**

EP 0 233 279 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, einer Blutpfropfbildung (Thrombose) in gewissem Umfang durch Verabreichung gerinnungshemmender Arzneimittel entgegenzuwirken. Gerinnungshemmende Arzneimittel (Antikoagulantien) im engeren Sinne verzögern oder verhindern die Blutgerinnung; zu diesen Arzneimitteln zählen Heparin und die Cumarine, wie z. B. Marcumar® (3-(1-Phenyl-propyl)-4-hydroxycumarin). Daneben sind auch noch sogenannte gerinnungshemmende Arzneimittel im weiteren Sinn bekannt, das sind bestimmte fibrinolytische und thrombolytische (d. h. fibrin- und blutpfropfauflösende) Stoffe wie z. B. Streptokinase und Urokinase, und Hemmstoffe der Thrombocytenaggregation bzw. der Prostaglandin-Synthese, wie z. B. Acetylsalicylsäure.

Taurolin wird als gut bakterizid wirksame Substanz in der Medizin verwendet. Taurolin weist eine geringe Löslichkeit in Wasser auf (ca. 1 %). Aufgrund seiner sehr guten bakteriziden und übrigen Eigenschaften (keine Resistenz gegen diese Substanz bekannt; außer vereinzelt auftretenden geringfügigen lokalen Gewebsirritationen sind andere unerwünschte Nebenwirkungen nicht bekannt) werden Lösungen von Taurolin für Knochenspülungen zur Bekämpfung und Behandlung der bakteriziden Osteolitis und bakteriellen eitrigen Peritonitis verwendet. Neben der sehr guten bakteriziden Wirksamkeit ist für Taurolin noch eine hemmende Wirkung auf die Ausbildung von Verklebungen des Bauchfells nach operativen Eingriffen in der Bauchhöhle bekannt (FR-A-2 455 890), sowie die Eignung von Taurolin zur Neutralisation des Endotoxins beim sogenannten Endotoxin-Schock; weitere medizinische Anwendungsgebiete sind für Taurolin bisher nicht bekannt (vgl. z. B. C. Steinbach-Lebbin et al, Arzneimittel-Forschung/Drug Research 32 (II), Nr. 12 (1982), 1542-1546; W. Siegenthaler, Klinische Pathophysiologie, Georg Thieme Verlag, Stuttgart 1976).

Es wurde nun gefunden, daß Taurolin neben seiner bekannten guten bakteriziden Wirkung überraschenderweise auch eine gerinnungshemmende Wirkung aufweist. Gegenstand der vorliegenden Erfindung ist deshalb die Verwendung von Taurolin zur Herstellung von Mitteln für die Hemmung der Blutgerinnung, insbesondere im extrakorporalen Kreislauf, beispielsweise bei Plasmaphorese, Dialyse, Blutwäsche, Oxigenatoren und bei Prothesen im Gefäßsystem.

Die Wirkung gemäß der Erfindung ist überraschend und noch in "Taurolin", herausgegeben von W.L. Brückner und R.W. Pfirrmann, Verlag Urban und Schwarzenberg, München 1985, wird ausdrücklich festgestellt, daß Taurolin die Blutgerinnung nicht beeinflußt und keine antiphlogistische Wirkung aufweist.

Da die gerinnungshemmende Wirkung von Taurolin sowohl in vivo als auch in vitro auftritt, kann Taurolin, wie erwähnt, auch zur Gerinnungshemmung im extrakorporalen Kreislauf (z. B. bei Herzoperationen oder bei der Hämodialyse in der künstlichen Niere) verwendet werden. Die Anwendung des Taurolins ist dabei vor allem in solchen Fällen angezeigt, in denen die Bildung oder Verschleppung von Blutpfropfen im Gefäßsystem (z. B. Thrombose Embolie) verhindert werden soll, also in erster Linie beim Herzinfarkt und bei Venenthrombose mit der Gefahr einer Lungenembolie, aber auch bei drohenden Verschlüssen von Blutgefäßen, bei Blutgefäßerkrankungen und nach Verletzungen oder Operationen zur Verhinderung der posttraumatischen Thrombosen.

Eine unerwünschte Nebenwirkung der bekannten Antikoagulantien, wie Heparin oder der Cumarinderivate, resultiert vor allem aus ihren erwünschten gerinnungshemmenden und daher u. U. blutungsfördernden Wirkungen. Es kann dabei vor allem zu Blutungen im Magen-Darm-Kanal (z. B. beim sog. "stillen" Magengeschwür), zu Hirnblutungen und zu Blutungen in die ableitenden Harnwege oder zu Wundblutungen während und nach Operationen und nach Verletzungen kommen. Zur Vermeidung derartiger unerwünschter Erscheinungen ist es deshalb wichtig, die verbleibende Gerinnungsfähigkeit des Blutes während der Verabreichung durch verschiedene Gerinnungstests, z. B. durch den sog. "Quick-Test", zu überwachen. Im Falle einer Überdosierung mußte dann bisher die zu starke Wirkung bei den Cumarinderivaten durch hohe Dosen von Gerinnungsfaktoren und gegebenenfalls auch Vitamin K reduziert werden, und die zu starke Wirkung von Heparin muß z. B. durch Neutralisation mit Protamin aufgehoben werden.

Es wurde nun festgestellt, daß bei der erfindungsgemäßen Verwendung von Taurolin als gerinnungshemmendes Mittel diese Nachteile nicht auftreten, sondern vielmehr immer noch eine ausreichende Blutgerinnungsfähigkeit erhalten bleibt und deshalb selbst bei einer versehentlichen Falschdosierung nicht die Gefahr besteht, daß eine völlige Unterdrückung der Blutgerinnung erfolgt und damit gefährliche Blutungen auftreten können. So wurde z. B. in vitro gefunden, daß bei der Zugabe von Taurolin zu Blutplasma (5 ml gepooltes Plasma verschiedener Personen wurde mit steigenden Mengen, bis max. 50 mg Taurolin, versetzt, wobei die Maximaldosis, d. h. also 50 mg/5 ml einer 1%igen Lösung entsprechen, was gleichzeitig auch der Löslichkeitsgrenze des Taurolins entspricht) eine entsprechende Verringerung des quick-Wertes und eine ebenfalls entsprechende Verlängerung der

PTT-Zeit auftritt und die Blutgerinnung nicht völlig unterdrückt wird, wie dies z. B. unter den gleichen Bedingungen bei Verwendung von Heparin der Fall ist. Die Verwendung von Taurolin als Gerinnungshemmer hat deshalb gegenüber bekannten Antikoagulantien den Vorteil, daß immer noch eine ausreichende Blutgerinnungsfähigkeit erhalten bleibt und deshalb eine Überdosierung nicht möglich ist.

Bei Gefäßprothesen wurde bisher die Thrombenbildung an der Oberfläche entweder durch Bindung von gerinnungshemmendem Heparin in die Innenwand der Prothesen oder neuerdings zunehmend durch Beschichtungen mit physikalischen Plasmen verhindert. Die Wirkung dieser Maßnahmen stellte sich jedoch als sehr kurzfristig heraus. Für die langfristige Aufrechterhaltung der Funktion einer Gefäßprothese strebt man nun die Bildung einer sogenannten Neointima auf den Innenwänden von Prothesen an. Zur Bildung einer Neointima müssen die Kunststoffoberflächen des Lumens der Gefäßprothese (z. B. Dacron® oder Teflon®) veloursartig gewebt vorliegen und mit Blutbestandteilen in Berührung gebracht werden; dies geschieht am besten bereits vor der Einpflanzung. Nach der Einpflanzung erfolgt dann eine Ablagerung von weißen Blutzellen auf den so vorbehandelten Oberflächen. Nach einem längeren Kontakt mit dem Blut ergab sich die Ausbildung einer weitgehend geschlossenen Endothelschicht auf der Veloursoberfläche. Diese künstlich erzeugte Endothelschicht, die als Neointima bezeichnet wird, kann die unerwünschte Thrombenbildung auf der Oberfläche der Prothesen verhindern. Dieses Verfahren besitzt aber den Nachteil, daß es sehr zeitaufwendig und umständlich ist. Außerdem ist es dabei erforderlich, bis zur Ausbildung einer zuverlässigen Neointima über Monate und Jahre zusätzlich systemisch Antikoagulantien zu verabreichen, was mit den bekannten Risiken verbunden ist.

Bei der erfindungsgemäßen Verwendung von Taurolin zur Herstellung von Gerinnungshemmern kann dieser Nachteil vermieden werden, d. h. das zeitaufwendige und umständliche Verfahren zur Verhinderung der Thrombenbildung auf der Prothesenoberfläche kann auf einfache Weise dadurch umgangen werden, daß man die Oberfläche mit Taurolin behandelt und dann direkt dem Blutstrom aussetzt. Auf diese Weise wird das Ergebnis, das man sonst durch Bildung der Neointima erzielt, praktisch sofort erreicht. Alternativ kann man das Taurolin auch direkt in der Blutstrom infundieren. Auch diese Ausführungsform ist unbedenklich, weil das Taurolin bei der maximal möglichen Dosierung im Blut den Quick-Wert bis maximal 20 % erniedrigt und den PTT-Wert nicht über 60 Sekunden anhebt. Diese Daten entsprechen den therapeutischen Zielwerten bei der Behandlung von Herzinfarktpatienten mit Marcumar®. Dies bedeutet, daß man also auch bei der Behandlung des Herzinfarktes die bisher bekannten Antikoagulantien, wie z. B. Marcumar® und Heparin, durch Taurolin ersetzen kann, wobei man außerdem auch noch die mit den bisher bekannten Antikoagulantien verbundenen Nachteile, wie insbesondere die Gefahr von Blutungen, vermeiden kann.

Die Dosierung und Anwendungsweise von Taurolin richtet sich je nach Anwendungsgebiet und therapeutischer Zielsetzung im allgemeinen nach den für die bekannten Antikoagulantien, wie z. B. Marcumar® und Heparin bekannten Richtlinien, wobei die für den jeweiligen Einzelfall geeignetsten Werte, Verabreichungsarten und Maßnahmen durch orientierende Tests, wie z. B. die Messung der Verringerung des Quick-Wertes, leicht zu bestimmen sind. Als Taurolin werden im Rahmen der Erfindung Verbindungen der allgemeinen Formel

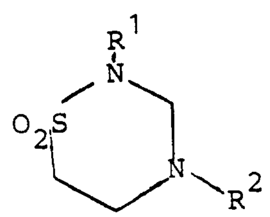

worin

$R^1$ ein Wasserstoffatom oder einen Alkylrest mit 1 bis 6 Kohlenstoffatomen und

$R^2$ ein Wasserstoffatom, einen Alkylrest mit 1 bis 6 Kohlenstoffatomen oder einen Rest gemäß der allgemeinen Formel

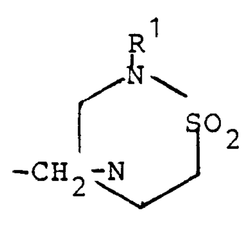

worin

$R^1$ die oben angegebenen Bedeutungen besitzt, bedeuten, verstanden.

Bevorzugt wird (4,4'-Methylen-bis(tetrahydro-2H-1,2,4-thiadiazin-1,1-dioxid)). Eine gleichartige gerinnungshemmende Wirkung, zum Teil in abgeschwächter Form, können auch die in einer wäßrigen Lösung von Taurolin damit im Gleichgewicht stehenden Verbindungen (z. B. 2H-1,2,4-Thiadiazin-1,1-dioxid und insbesondere sein 4-Hydroxymethyl-Derivat, und die beim Erhitzen im Gleichgewicht stehenden Ringspaltungsprodukte, wie z. B. 1-Hydroxymethylamino-2-sulfamoyläthan und 1-Amino-2-sulfamoyl-äthan), sowie die Taurolin-Metaboliten, zeigen (vgl. C. Steinbach-Lebbin et al, Arzneimittel-Forschung/Drug Research 32 (II), Nr. 12 (1982) 1542-1546). Unter Taurolin sind vorstehend und nachstehend deshalb auch diese Abbauprodukte und Metaboliten zu verstehen.

Als Dialysematerialien uni Gefäßprothesen können alle bisher bekannten und in der Literatur beschriebenen Materialien verwendet werden (vgl. z. B. Artificial Organs, Vol. 5 (Suppl) 1981 507; Trans. Am. Soc. Artif. Intern. Organs, Vol. XXV (1979) 280; Vol. XXVII (1981) 396, 499, 511 und 648; Vol. XXVIII (1982) 459 und 478; Vol. XXIX (1983) 200).

Die Verabreichung kann in der Regel in der für Antikoagulantien üblichen Art und Weise erfolgen, also z. B. intravenös, intraperitoneal, peroral oder als Infusion. Gegenüber Heparin besitzt das erfindungsgemäß verwendete Taurolin weiters den Vorteil, daß es die Wirkung bei gleich schnellem Eintritt lange und gleichmäßig beibehält und Taurolin auch oral verabreicht werden kann; dadurch ist die bisher in vielen Fällen erforderliche Anwendung (z. B. bei der Behandlung eines Herzinfarktes) der Kombination einer anfänglichen Heparininjektion und nachfolgender oraler Gabe eines Cumarinderivats, dessen Wirkung zwar verzögert einsetzt, dafür aber in oraler Form appliziert werden kann, nicht erforderlich. Bevorzugt wird eine Lösung von Taurolin in Wasser angewendet.

Zur erfindungsgemäßen Verwendung zur Herstellung gerinnungshemmender Mittel kann das Taurolin als alleinige Komponente, oder auch zusammen mit anderen Bestandteilen verabreicht werden. Die Verabreichungsform (pharmazeutische Zubereitung) kann übliche Zusatzstoffe, wie übliche pharmazeutische Konfektionierungs- und/oder Verdünnungsmittel enthalten, und gegebenenfalls noch weitere Wirkstoffe, sofern diese mit den übrigen Bestandteilen keine unerwünschten Nebenwirkungen zeigen und zur Unterstützung der Therapie geeignet sind; insbesondere kann es in einigen Fällen auch zweckmäßig sein, das Taurolin zusammen mit bekannten Antikoagulantien, wie z. B. Heparin und/oder Cumarinderivaten, wie Marcumar® anzuwenden, z. B. gleichzeitig (in der gleichen Verabreichungsform), oder auch hintereinander. Hierdurch ist auch eine Verringerung der Dosis der bekannten Antikoagulantien und damit ihrer bekannten Anwendungsrisiken möglich.

Es wurde auch gefunden, daß der einmalige Kontakt mit Taurolin mit einer künstlichen Oberfläche, wie z. B. einer Kunststoff- oder Metalloberfläche, die Abscheidung von Blutgerinnseln auf der künstlichen Oberfläche in vivo, d. h. nach einer Einpflanzung oder einer vorübergehenden Einführung des Gegenstandes aus dem künstlichen Material in einen blutdurchströmten Organismus, dauerhaft wirksam verhindert. Diese gerinnungshemmende Wirkung kann deshalb zur Verwendung des Taurolins zur Behandlung der Oberflächen von medizinischen Geräten, die vorübergehend in die Blutbahn eingeführt werden, wie z. B. Venenkatheter (die unbehandelt zur Verstopfung durch Blutkoagel neigen, was wieder einen Nährboden für Bakterien darstellt, wodurch die Gefahr einer Sepsis hervorgerufen wird) dienen. Die Behandlung der Geräte kann z. B. mit einer wäßrigen Taurolinlösung, insbesondere einer 1%igen wäßrigen Taurolinlösung, durchgeführt werden. Durch eine intermittierende Spülung von Gefäßkathetern ist es auf diese Weise z. B. möglich, die Koagelbildung an den Katheterspitzen zu verhindern.

Die Wirkungsweise von Taurolin auf das Blutgerinnungssystem unterscheidet sich grundsätzlich von der der bisher bekannten gerinnungshemmenden Mittel. Cumarin-Derivate wirken durch Verminderung der Syntheserate von Gerinnungsfaktoren - als Vitamin K-Antagonisten -; daher tritt ihre Wirkung sehr verzögert auf, d. h. in Abhängigkeit von der Halbwertszeit der Gerinnungsfaktoren, die zwischen 20 und 100 Stunden beträgt. Heparin wirkt durch Aktivierung des Antithrombin-III, das seinerseits als Hemmer der Serinproteasen des Gerinnungssystems fungiert; es wird weiterhin an die Zelloberflächen der wandständigen Zellen der Blutgefäße - die Endothelzellen - und an verschiedene Co-Faktoren der Gerinnung gebunden. Im Gegensatz dazu hemmt Taurolin konzentrationsabhängig und damit gut steuerbar die Funktion des Antithrombin-III. Dadurch kann Taurolin auch als Antidot (Gegenmittel) bei Heparin-Überdosierungen eingesetzt werden, mit dem Vorteil, daß nach der Anwendung von Taurolin durch das Taurolin weiterhin eine gerinnungshemmende Wirkung bestehen bleibt.

Aufgrund seiner überraschenden neuartigen gerinnungshemmenden Wirkung kommt weiters auch die Verwendung von Taurolin als Antidot (Gegenmittel) auch dann in Frage, wenn durch andere in den Organismus gelangte blutgerinnungswirksame Substanzen, wie z. B. Schlangengifte, das Gerinnungssystem aktiviert wurde (vgl. z. B. "Methods in Enzymology", Vol. XLV, "Proteolytic Enzymes" Teil B, 1976, Academic Press, New York/San Francisco/London).

Wie die gerinnungshemmende Wirkung des Taurolins zustande kommt, konnte noch nicht völlig geklärt werden. Jedoch wurde eine ausgeprägte direkte Wirkung des Taurolins auf den Faktor XII des Gerinnungssystems festgestellt; dies ist deshalb von besonderem Interesse, weil der aktivierte Faktor XIIa die Aktivierung des kallikrein/Kinin-Systems, des Fibrinolysesystems und des Komplementsystems bewirkt. Insbesondere werden über das Kallikrein/Kinin-System, aber auch über das Komplement-System, Entzündungsreaktionen

des Gefäß-Bindegewebes hervorgerufen.

Die nachfolgenden Beispiele erläutern die Erfindung näher.

**Beispiel 1**

Folien aus Zelluloseacetat wurden mit einer 1%igen wäßrigen Lösung von Taurolin (Einwirkungszeit 30 Minuten bei 37°C) behandelt und anschließend mit Frischblut in Berührung gebracht (Einwirkungszeit 30 Minuten bei 37°C). Nach Entfernung des Blutes und Abspülen der Folienoberfläche mit physiologischer Kochsalzlösung konnte durch Elektronenraster-Fotografien keine Fibrinfaserabscheidung auf der Oberfläche festgestellt werden, während eine solche bei sonst gleichen, aber nicht mit Taurolinlösung vorbehandelten Oberflächen festgestellt wurde.

Eine Zelluloseacetat-Folie, die zunächst mit einem Taurolin/Blutplasma-Gemisch (Taurolin 1%ig im Plasma gelöst) in Berührung gebracht wurde, zeigt anschließend auch dann keine wandständige Blutgerinnung oder Fibrinablagerung, wenn sie danach mit physiologischer Kochsalzlösung gespült und dann mit Frischblut von gesunden Spendern 30 Minuten bei 37°C inkubiert wird.

Es wurde auch gefunden, daß ganz allgemein die Oberflächen von künstlichen Materialien, die in einen Organismus implantiert werden, durch Kontakt mit Taurolin thromboresistent werden und dieser Effekt auch dann anhält, wenn das Taurolin bereits von der Oberfläche entfernt wurde. Hieraus ist zu erkennen, daß der einmalige Kontakt von Taurolin mit einer künstlichen Oberfläche die Abscheidung von Blutgerinnseln auf der künstlichen Oberfläche in vivo, d. h. nach Einpflanzung der Vorrichtung aus künstlichem Material in einen blutdurchströmten Organismus, dauerhaft wirksam verhindert.

**Beispiel 2**

Proben von jeweils 5 ml eines gepoolten Plasmas verschiedener Versuchspersonen wurden mit steigenden Mengen an Taurolin versetzt, und zwar mit Mengen von 0 (1), 10 (2), 20 (3), 30 (4), 40 (5) und 50 (6) mg Taurolin/5 ml Plasma (die in Klammern stehenden Ziffern geben die Probennummern an).

Für die Proben 1 bis 6 wurde der Quick-Test und der PTT-Test durchgeführt und die folgenden Ergebnisse (30 Minuten) erhalten (Tabelle 1):

| Proben-Nr. | 1 | 2 | 3 | 4 | 5 | 6 |
|---|---|---|---|---|---|---|
| Quick-Test (%) | 92 | 65 | 53 | 44 | 39 | 35 |
| PTT (in sec) | 34,1 | 38,0 | 43,0 | 48,8 | 55,9 | 61,8 |

Außerdem wurde bei den Proben 1 bis 6 für die verschiedenen Gerinnungsfaktoren (Plasmafaktoren) die Herabsetzung der Werte durch die Taurolinzugabe bestimmt (nach den Methoden der Faktorenanalyse, wie sie von Herstellern entsprechender Testsysteme, z. B. von Behringwerke AG, Marburg oder American Hospital Supply Deutschland GmbH, München, angegeben werden.)

Die erhaltenen Ergebnisse sind in Tabelle 2 zusammengestellt:

Tabelle 2

| Proben-Nr. | Gerinnungsfaktor II | V | VII | X | IX | XI | XII | VIII |
|---|---|---|---|---|---|---|---|---|
| 1 | 108% | 164% | 94% | 82% | 321% | 312% | 185% | 121% |
| 2 | 96% | 121% | 79% | 75% | 153% | 301% | 177% | 48% |
| 3 | 87% | 110% | 74% | 65% | 81% | 197% | 77% | 21% |
| 4 | 90% | 92% | 74% | 56% | 63% | 140% | 64% | 13% |
| 5 | 75% | 81% | 77% | 53% | 65% | 83% | 55% | 6% |
| 6 | 74% | 79% | 73% | 50% | 68% | 47% | 61% | 6% |

Die Werte der Tabelle 2 zeigen klar, daß sämtliche untersuchten Blutfaktoren durch das Taurolin vermindert werden (anfänglicher Wert ist der für die unbehandelte Probe Nr. 1 angegebene absolute Wert in Prozent), daß aber in keinem Fall eine Verringerung bis auf 0% stattfindet.

Die im Beispiel 2 erhaltenen Ergebnisse zeigen, daß unter den angewendeten Bedingungen die Blutgerinnung durch das Taurolin nicht völlig unterdrückt wird, während dies z. B. beim Ersatz von Taurolin durch Heparin unter sonst gleichen Bedingungen der Fall ist. Daraus ist ersichtlich, daß das erfindungsgemäß verwendete Taurolin bei seiner Anwendung als Gerinnungshemmer weniger bedenklich ist als die bisher bekannten Gerinnungshemmer, weil durch die nicht vollständige Unterdrückung der Blutgerinnung auch bei einer unbeabsichtigten Falschdosierung (Überdosierung) nicht die Gefahr einer vollständigen Unterdrückung der Blutgerinnung, und damit einer Blutung, besteht.

## Patentansprüche

1. Verwendung von Taurolin zur Herstellung von Mitteln für die Hemmung der Blutgerinnung.
2. Verwendung von Taurolin zur Herstellung von Mitteln für die Hemmung der Blutgerinnung im extrakorporalen Kreislauf.
3. Verwendung von Taurolin zur Herstellung von Mitteln für die Hemmung der Blutgerinnung in Gefäßprothesen.
4. Verwendung von Taurolin zur Herstellung von Mitteln, welche als Antidot von blutgerinnungswirksamen Mitteln wirken.

## Claims

1. Use of taurolin for the production of agents for the inhibition of blood coagulation.
2. Use of taurolin for the production of agents for the inhibition of blood coagulation in the extracorporeal blood circulation.
3. Use of taurolin for the production of agents for the inhibition of blood coagulation in vascular protheses.
4. Use of taurolin for the production of agents which act as antidote of blood coagulation-active agents.

## Revendications

1. Utilisation de la tauroline pour la préparation d'agents pour inhiber la coagulation du sang.
2. Utilisation de la tauroline pour la préparation d'agents pour inhiber la coagulation du sang dans une circulation extracorporelle.

3. Utilisation de la tauroline pour la préparation d'agents pour inhiber la coagulation du sang dans des prothèses de vaisseaux.

4. Utilisation de la tauroline pour la préparation d'agents qui agissent comme antidote d'agents actifs pour la coagulation du sang.